# EUROPEAN PATENT APPLICATION

(11) **EP 1 535 579 A2**
(43) Date of publication of application: **01.06.2005**
(21) Application number: 04257314.7
(22) Date of filing: 25.11.2004
(51) Int. Cl.: A61B 17/16

(54) **Expandable reamer**

(30) Priority: 25.11.2003 US 721808
(71) Applicant: Zimmer Technology, Inc., Chicago, Illinois 60606 (US)
(72) Inventor: Lozier, Antony J., Warsaw, IN 46582 (US); Pacelli, Nicolas J., Winona Lake, IN 46590 (US); Thelen, Sarah L., North Manchester, IN 46962 (US)
(74) Representative: Mays, Julie

(57) **Abstract**

An expandable reamer includes, in one exemplary embodiment thereof, a cannulated shaft and a plurality of straight cutting blades having deformable points. The blades are hingably outwardly rotatable at the deformation points between a contracted position and an expanded position. In the contracted position, the blades are substantially parallel to the longitudinal axis of the cannulated shaft and, in the expanded position, the blades have at least a portion oriented radially outward from the longitudinal axis, thereby forming a larger diameter cutting surface in the expanded position and in the contracted position. The blades are formed from a portion of the cannulated shaft by, e.g. milling longitudinally extending slots through the wall of the cannulated shaft, the slots serving as flutes dividing the cutting edge and trailing edge of each adjacent blade. Each blade may also include more than one segment arranged along its length, the segments coupled by deformation points. The expandable reamer may be used for cutting a cavity in a bone or other structure that is larger than the diameter of the entry point into the bone and greater than the diameter of the contracted reamer.

## Description

### BACKGROUND

The present invention relates to reamers and, more specifically, to reamers having expandable reaming heads.

Reamers are typically used for enlarging the diameter of a bore which has been drilled or otherwise cut in a material. Reamers generally include a shank for driving the reamer and a reamer body that includes cutting edges. Hand or powered rotation of a reamer cuts or shaves the material surface defining the bore, removing material and increasing the diameter of the bore.

Certain reaming applications require the reaming of a cavity that is larger in diameter than an aperture allowing access to the cavity. One known expandable reamer used for spinal surgical procedures provides an elongated shaft assembly having a pair of opposing blades rotatably mounted in a scissor-like fashion at the distal end of the shaft assembly. After insertion of the distal end of the shaft assembly through an aperature leading to a bore in a bone structure, the blades may be rotated radially outwardly to increase the effective cutting diameter of the reamer. After reaming a cavity of the desired size, the reamer blades may be rotated to a position in which the outer diameter of the blades is less than the aperature diameter to allow for withdrawal of the reamer from the bone structure.

Orthopedic procedures for the replacement of all, or a portion of, a patient's joint generally require an open procedure wherein an incision is made through the skin and the underlying muscle and other tissue to fully expose the relevant joint. While this approach provides surgeons with an excellent view of the bone surface and open access for various sized and shaped instruments such as cutting and reaming instruments, the underlying damage to the soft tissue, including the muscles, can lengthen a patient's healing and rehabilitation time after surgery. Therefore, it is desirable to minimize the size of the incision and the damage to the underlying muscle.

What is needed in the art is a method and device for reaming bone cavities which are larger than the incision of the soft tissue and/or aperture into the bone, and without requiring expensive and separate boring and reaming instruments.

### SUMMARY OF THE INVENTION

The present invention provides a method and device for cutting a cavity in a structure, the cavity having a greater diameter than the aperture providing access to the cavity. The method and device of the present invention may be used, for example, for cutting a cavity in a bone structure using minimally invasive surgical procedures, for example, for performing a minimally invasive total hip arthroplasty.

An exemplary embodiment of an expandable reamer according to the present invention may include a cannulated shaft and a plurality of straight cutting blades coupled to the cannulated shaft and having deformation points. The blades of this form of the present invention are outwardly deformable between a contracted position and an expanded position. In the contracted position, the blades are substantially parallel to the longitudinal axis of the cannulated shaft and, in the expanded position, the blades have at least a portion oriented radially outward from the longitudinal axis of the cannulated shaft, thereby forming a larger diameter cutting surface in the expanded position than in the contracted position.

The blades may be formed from a portion of the cannulated shaft by milling, etching, stamping, or otherwise forming longitudinally oriented slots through the wall of the cannulated shaft, the slots serving as flutes dividing the cutting edge and trailing edge of each adjacent blade. Each blade may be segmented along its length, the segments separated at a point of deformation. The location of deformation points provide a desired shape to the cutting surfaces when the reamer body is placed in the expanded position.

The reamer may be expanded by drawing the distal end of the reamer blades toward the proximal end of the blades, and may be contracted by advancing the distal end of the blades away from the proximal end of the blades. Advantageously, the expandable reamer may be used for cutting a cavity in a bone or other structure that is larger than the diameter of the soft tissue incision and aperture into the bone and greater than the diameter of the contracted reamer.

In one exemplary embodiment, an expandable reamer of the present invention includes a cannulated shaft defining a shank and a reamer body. The reamer body defines a plurality of blades having longitudinally extending slots therebetween and an end cutter disposed at the distal end of the reamer body. Distal ends of the blades may be coupled to a ring on which the end cutter is positioned. Proximate ends of the blades are coupled to the shank. The blades may be deformable at the point of coupling with the ring and shank. The length of the blade may be divided into two or more segments, the segments separated by a deformation point.

By proximally drawing the ring and distal end of the blades toward the proximate end of the blades, deformation of the blades at the deformation points allows the segments to extend radially outward from the longitudinal axis of the reamer, thereby increasing the diameter of the reamer body. Distally advancing the distal ring along the longitudinal axis away from the proximate end of the blades will cause the blades to contract radially inward toward the longitudinal axis, thereby returning the reamer body to its original diameter and the blades to a contracted position substantially parallel to the longitudinal axis of the reamer.

In one exemplary embodiment, the deformation points at which the blades are coupled to the distal ring and to the shank and which separate adjacent blade segments may be defined simply by exterior or interior circumferential reliefs or grooves which reduce the material thickness and therefore reduce resistance of the blades to bending at the various desired points. The deformation points may also be further defined by radially oriented arcuate cuts which intersect the circumferential reliefs.

In one exemplary embodiment of the invention, the blades are easily and inexpensively formed from a reamer body having a polygonal cross-section, such as a hexagon. The slots may be milled parallel to and coincident with the apex formed between adjacent sides of the polygon. By locating the slots in this way, each apex and the milled face which extends radially inward form cutting edges, and the opposite milled face of the slot forms the trailing edge, or flute, of an adjacent blade. Formed in this fashion, the cutting edge, being the former apex of the polygon, has a greater radius than the trailing edge. Thus, only the cutting edge contacts the surface being reamed.

The expandable reamer of the present invention is an inexpensive and possibly disposable device. The deformation points of the reamer body of the present invention can be positioned to form predefined complex shapes for boring and reaming a cavity in a bone as part of a minimally invasive orthopedic surgery. Such procedures include, for example, those disclosed in "Method and Apparatus for Reducing Femoral Fractures," U.S. Patent Application Serial No. 10/155,683, filed May 23, 2002; U.S. Patent Application Serial No. 10/266,319, filed October 8, 2002; U.S. Patent No. 10/358,009, filed February 4, 2003; and "Method and Apparatus for Performing a Minimally Invasive Total Hip Arthroplasty," U.S. Patent Application Serial No. 09/558,044, filed April 26, 2000; U.S. Patent Application Serial No. 09/992,639, filed November 6, 2001, and published as U.S. Publication No. US 2002/0099447 A1; U.S. Patent Application Serial No. 10/053,931, filed January 22, 2002, and published as U.S. Publication No. US 2002/0116067 A1, on August 22, 2002, and U.S. Patent No. 10/357,948, filed February 4, 2003; the disclosures of which are hereby incorporated by reference herein.

In order to ream a cavity in a bone that is larger than the incision in the soft tissue and the entry aperture into the bone, the expandable reamer is first inserted through the incision and the aperture in the bone. Then, the reamer is expanded during rotation by drawing a distal end of the reamer body toward the proximate end of the reamer body. Upon achieving the desired expansion diameter and thereby cavity size, the distal end of the expandable reamer may be advanced away from the proximate end of the reamer body, thereby collapsing the diameter of the expandable reamer so that it may be removed from the cavity and withdrawn through the entry aperture and incision.

Other embodiments of the expandable reamer are also envisioned. One such embodiment includes a reamer having blades that are uncoupled at a distal end, thus providing a larger cavity diameter at the distal end of the cavity. Another embodiment includes reamer blades that are flexibly biased to the expanded position, thereby providing a reamer that will expand and cut less dense or cancellous bone while contracting away from more dense cortical bone. Yet another embodiment expands to one of various predefined shapes according to the blade segment length and deformation members coupling the blade segments.

In one embodiment, a reamer according to the present invention includes a shank, a reamer body having a longitudinal axis, and a blade formed in said reamer body, the blade deformable between a contracted position and an expanded position.

In another embodiment, a reamer according to the present invention includes a shank, a reamer body having a longitudinal axis, a blade formed in the reamer body, and deformation means for deforming the blade between a contracted position and an expanded position.

In another embodiment, a reamer according to the present invention includes a cannulated shaft having a wall, a proximate end and a distal end and defining a longitudinal axis, the cannulated shaft having a plurality of slots therethrough, the plurality of slots extending from the distal end toward the proximate end, and a plurality of blades, each one of the plurality of blades defined by the wall between adjacent ones of the plurality of slots.

In yet another embodiment, a method of reaming a cavity in a bone according to the present invention includes providing an expandable reamer having blades moveable between a contracted position and an expanded position, boring an opening in the bone, the opening having a diameter at least as large as a diameter of the expandable reamer in a contracted position, inserting the expandable reamer into the opening, the expandable reamer being in the contracted position, rotating the expandable reamer while moving the blades to the expanded position, contracting the expandable reamer to the contracted position, and removing the expandable reamer from the cavity.

Advantageously, the present invention provides a low-cost and potentially disposable reamer that provides a predefined reamer body shape which is expandable after insertion into the bone structure, which includes deformable blades that are secured at both a distal and a proximate end, and which may include a distal end cutter for boring the initial bore into the bone structure.

### BRIEF DESCRIPTION OF THE DRAWINGS

The above mentioned and other features and objects of this invention, and the manner of attaining them, will become more apparent and the invention itself will be better understood by reference to the following description of embodiments of the invention taken in conjunction with the accompanying drawings, wherein:
Fig. 1 is a perspective view of a first exemplary embodiment expandable reamer according to the present invention;
Fig. 2 is a cross-sectional view of the reamer of Fig. 1;
Fig. 3A is a perspective view of the reamer of Fig. 1 coupled with a driving apparatus, the reamer shown in a contracted position;
Fig. 3B is a partial perspective view of the reamer of Fig. 3A, shown in an expanded position;
Fig. 4 is a partial cross-sectional view of the reamer of Fig. 3A, shown in the contracted position;
Fig. 5 is a radial plan view of a second exemplary embodiment according to the present invention;
Fig. 6 is a partial cut-away anterior view of a femur with the reamer of Fig. 5 being employed in a minimally invasive surgical procedure for removing the neck and head of the femur; and
Fig. 7 is a partial perspective view of a third embodiment expandable reamer according to the present invention.

Corresponding reference characters indicate corresponding parts throughout the several views. Although the drawings represent embodiments of the present invention, the drawings are not necessarily to scale and certain features may be exaggerated in order to better illustrate and explain the present invention. The exemplification set out herein illustrates embodiments of the invention, in several forms, and such exemplifications are not to be construed as limiting the scope of the invention in any manner.

### DESCRIPTION OF THE INVENTION

The embodiments disclosed below are not intended to be exhaustive or limit the invention to the precise forms disclosed in the following detailed description. Rather, the embodiments are chosen and described so that others skilled in the art may utilize their teachings.

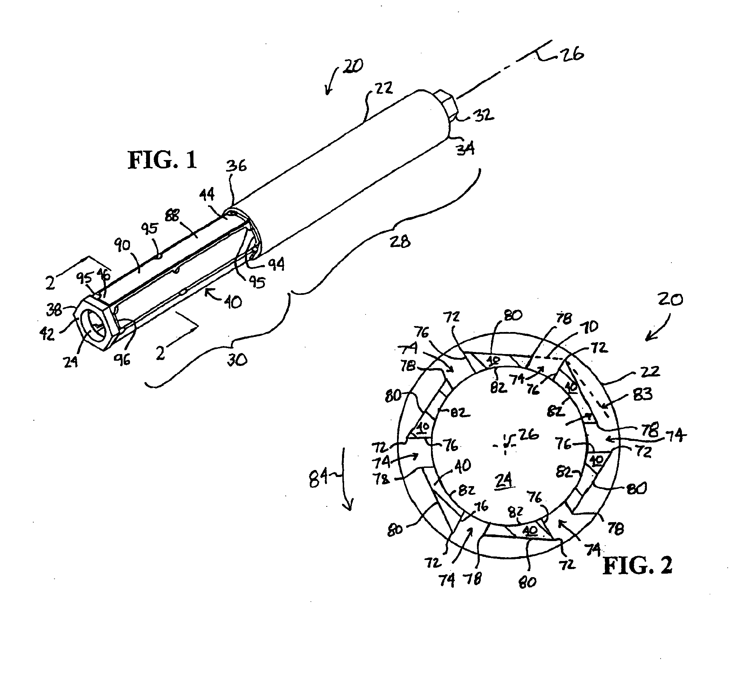

First exemplary expandable reamer 20, shown in Fig. 1, is formed from cannulated shaft 22 having bore 24 therethrough along longitudinal axis 26. Reamer 20 includes shank 28 and reamer body 30. Chuck end 32 for driving reamer 20 is located at proximate end 34 of shank 28. Reamer body 30 extending from distal end 36 of shank 28 to distal end 38 of reamer 20 includes deformable cutting blades 40 and ring 42. Blades 40 are coupled at proximate end 44 to distal end 36 of shank 28. Blades 40 are also coupled at distal end 46 to ring 42. Advantageously, as ring 42 is proximally drawn toward proximate end 32 of reamer 20, at least a portion of blades 40 extend radially outward from longitudinal axis 26, thereby increasing the cutting diameter of reamer 20.

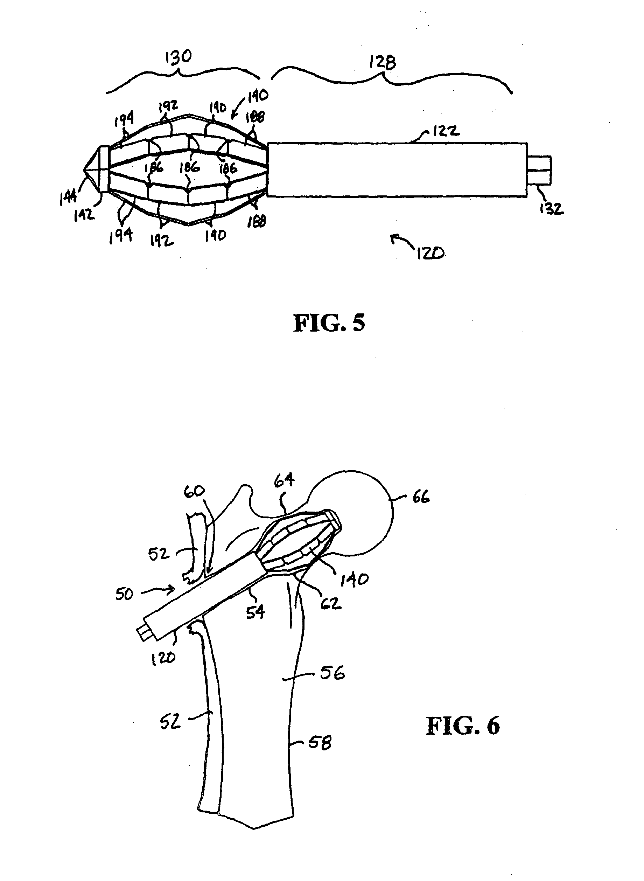

Expandable reamer 20 is useful for cutting a chamber or cavity in a structure, the cavity having a greater diameter than the entry aperture into the structure and a greater diameter than shaft 22. Referring to Fig. 6, for minimally invasive surgery, *e.g.*, total hip arthroplasty, expandable reamer 120 may be inserted through incision 50 defined in soft tissue 52 and through aperture 60 and into bore 54 drilled in bone structure 56 of femur 58. Optionally, to further protect soft tissue 52, a tubular retractor (not shown) may be inserted through incision 50, with reamer 120 inserted through the tubular retractor to prevent contact of soft tissue 52 with blades 140. In one exemplary embodiment, the tubular retractor is coupled to bone structure 56 and further functions to guide insertion of reamer 120.

After blades 140 of expandable reamer 120 are located with bore 54, blades 140 may be extended to an expanded position while rotating reamer 120, thereby forming cavity 62 in bone structure 56. For example, such a procedure using reamer 120 may be used to remove neck 64 and head 66 of femur 58.

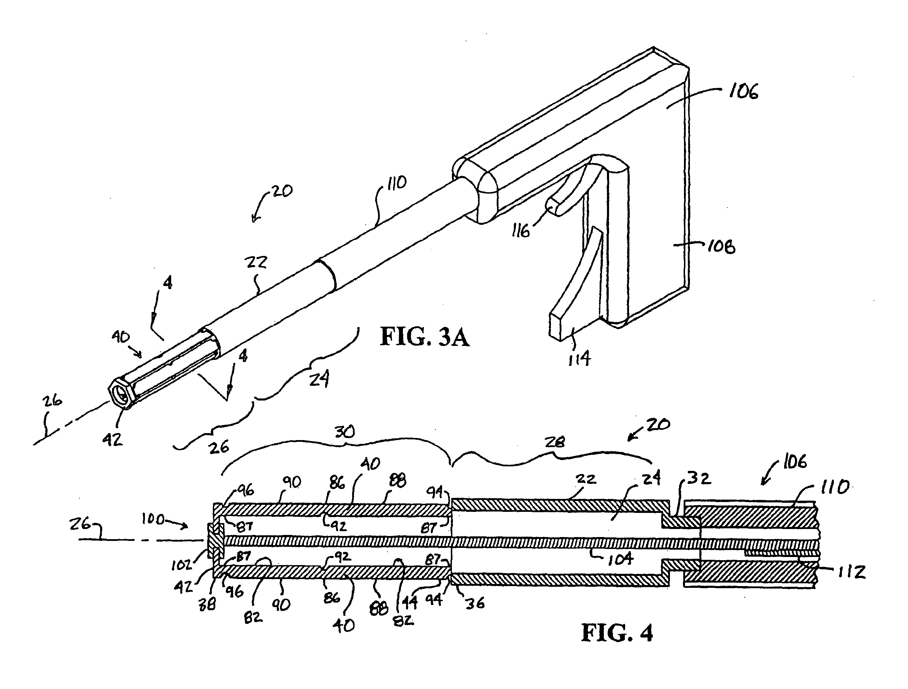

Referring to Figs. 1 and 2, first exemplary expandable reamer 20 includes six blades 40 and ring 42 which are formed from cannulated shaft 22. In this exemplary embodiment reamer body 30 has a hexagonal cross-section. The cross-section of reamer body 30 may be a circle or a different numbered polygon with the number of sides determining the number of resulting blades 40.

Referring to the cross-sectional end view shown in Fig. 2, blades 40 are formed by removing portion 70 which is coincident with one side of comer or apex 72 joining adjacent sides of the hexagon. Removing portions 70 creates slots 74 between adjacent blades 40. Slots 74 are milled in reamer body 30 so that face 76 is directed radially toward longitudinal axis 26. The wall of slot 74 opposite face 76 defines the trailing edge or heel 78 of the adjacently located blade 40. The remaining portion of the hexagonal side located between cutting edge 72 and heel 78, referred to as land 80, forms the outer surface of each blade 40. Inner surface 82 of blades 40 defines bore 24 formed through reamer body 30.

Because slot 74 is located coincident to two adjoining sides of the hexagonal shape of reamer body 30, cutting edge 72 has a greater radius relative to longitudinal axis 26 than any other point along land 80. Thus, clearance angle 83, defined as the difference in radius between cutting edge 72 and trailing edge 78, is provided so that cutting edge 72 of each blade 40 is the only portion of blades 40 that will be in contact with the material being reamed. First exemplary reamer 20 shown in Fig. 2 is formed for counterclockwise rotation 84, which rotates cutting edge 72 and face 76 toward the surface to be reamed.

Referring now to Figs. 1 and 4, outward radial expansion of portions of blades 40 is facilitated by deformation points 86. Each blade 40 is divided into proximal segment 88 and distal segment 90. Segments 88 and 90 are joined at deformation points 86. Deformation points 86 defined in blade 40 may be formed by interior circumferential relief 92 which is cut along interior surface 82 of each blade 40 and which reduces the resistance of blade 40 to bending or deforming outwardly.

Additionally, proximal segment 88 of each blade 40 is joined to shank 28. Deformation points 87 are formed by proximal exterior circumferential relief 94 cut in land 80 of each blade 40. Similarly, deformation points 87 are located in blade 40 where distal segment 90 of each blade 40 is connected to distal ring 42 of reamer body 30. Deformation points 87 may be formed by distal exterior circumferential relief 96 cut in each land of blade 40. Additionally, radially oriented arcuate notches 95 (Fig. 1) may be cut in blades 40 along cutting edge 72 and coincident with reliefs 92, 94, and 96, further reducing the resistance of blades 40 to bending to the expanded position. Although deformation points 86 and 87 are referred to as "points," deformation points 86 and 87 define lines or areas of deformation in blades 40.

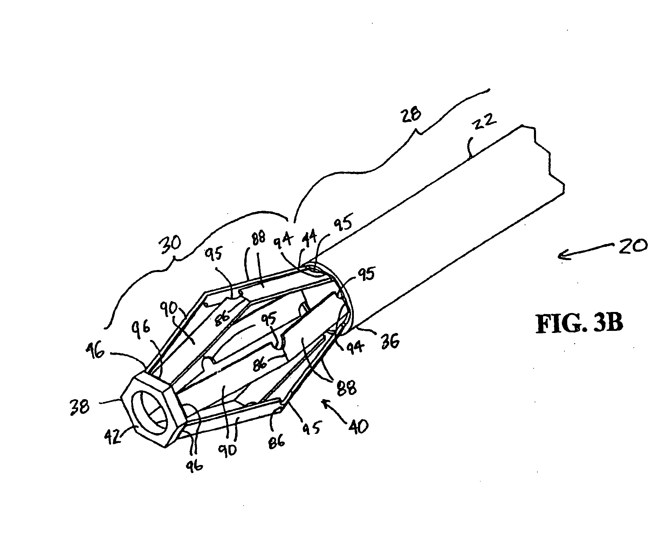

Referring to Fig. 3B, interior relief 92 and notches 95 are provided between segments 88 and 90 and exterior reliefs 94 and 96 and notches 95 are provided at the proximal end 44 of segment 88 and distal end 46 of segment 90. Reliefs 92, 94 and 96 and notches 95 facilitate folding or radially expanding the adjoining ends of segments 88 and 90 at interior relief 92 outwardly from longitudinal axis 26 and about proximate relief 94 and distal relief 96. Alternatively, other types of deformation points as are known in the art may be utilized to hingably connect blade segments 88 and 90. For example, a material or discrete member that is more easily deformable than blades 40 may be substituted at the points of deformation, the material at the points of deformation may be thinned or otherwise made pliable, or a hinge or other type of relative motion device or member may be utilized. If a point of deformation comprises a thinned region, then the cross-sectional area at the point of deformation is smaller than the cross-sectional area of the portions of the blade adjacent the deformation point.

Referring to Fig.4, mechanism 100 provides proximal translation of ring 42 toward distal end 36 of shank 28, thereby expanding blades 40 as described above. Mechanism 100 may comprise, for example, bushing 102, which may be rotatably coupled to ring 42, and elongate member 104, for example, a rod. Elongate member 104 extends through bore 24 in expandable reamer 20 and is operable to translate ring 42 along longitudinal axis 26. Exemplary driving device 106, shown in Figs. 3A and 4, may be utilized to rotationally drive reamer 20 and to longitudinally translate elongate member 104. Driving device 106 includes handle 108, rotational drive 110 and translational drive 112; however, other devices or mechanisms capable of effecting rotational and translational motion may be utilized.

As shown in Fig. 4, rotational drive 110 of driving device 106 may be coupled with chuck end 32 and translational drive 112 may be coupled with elongate member 104. Referring to Fig. 3A, first actuator 114 functions to rotate rotational drive 110 and thus reamer 20 about longitudinal axis 26. Second actuator 116 functions to translate translational drive 112 and thus elongate member 104 and bushing 102 along longitudinal axis 26. By actuating second actuator 116 in a first direction, bushing 102 is drawn toward distal end 36 of shank 28, thereby deforming blades 42 radially outwardly to the expanded position shown in Fig. 3B. Actuating second actuator 116 in a second direction distally advances elongate member 104 and bushing 102 away from distal end 36 of shank 28, thereby returning blades 40 to the contracted position, substantially parallel to longitudinal axis 26 as shown in Fig. 3A.

Referring to Fig. 5, second exemplary expandable reamer 120 includes cannulated shaft 122 defining shank 128, Chuck end 132, reamer body 130, distal ring 142, and end cutter 144. End cutter 144 may be secured to ring 142 and may be used as an end mill to cut the bore which reamer 120 may then ream into a larger diameter cavity.

Reamer body 130 includes blades 140 which are divided into multiple blade segments 188, 190, 192, and 194. Advantageously, the relative length and locations of segments 188-194 and deformation members 186 joining them may be designed to provide a specific desired shape and diameter of reamer 120 when in the expanded position shown in Fig. 5. For example, an exemplary reamer may have two short segments coupled to opposite ends of a central long segment, thus providing a long cutting surface of uniform diameter between the distal and proximal ends of the blades. An exemplary reamer may alternatively have a single segment formed from pliable material which bows outwardly between the proximal and distal ends when compressed.

Referring to Fig. 6, advantageously, expandable reamer 120 may be used to cut cavity 62 in bone structure 56 while maintaining minimally invasive surgical procedures. For example, incision 50 may be cut in soft tissue 52, a cylindrical sleeve (not shown) may be positioned through incision 50 to hold open incision 50 and protect soft tissue 52 from damage by reamer 120, and then a drill or end cutter 144 of expandable reamer 120, may be used to form aperture 60 and bore 54 in bone structure 56 of femur 58.

In certain orthopedic procedures, it is necessary to cut large diameter cavity 62 in bone structure 56, which may be, for example, femur 58. After bore 54 is formed in bone structure 56, reamer 120 may be inserted through incision 50 and aperture 60 into bore 54. While driven rotationally, blades 140 are expanded so that blades 140 ream bore 54 to an increased diameter, thus forming cavity 62. Cavity 62, having been formed by reamer 120 in an expanded position, has a larger diameter than the diameter of aperature 60 and incision 50. After cavity 62 is reamed to the desired diameter, blades 140 of reamer 120 may be contracted to their original diameter as described above, and reamer 120 removed through aperture 60 and incision 50. Debris from removed bone structure 56 may then be flushed or otherwise removed from cavity 62 in hole 64.

The inventive reamer may also be used for other procedures requiring reaming and cutting. For example, for a minimally invasive total hip arthroplasty, rather than cutting cavity 62, reamer 120 may be used, as above, to remove a complete portion of bone structure 56, for example, neck 64 and head 66 of femur 58.

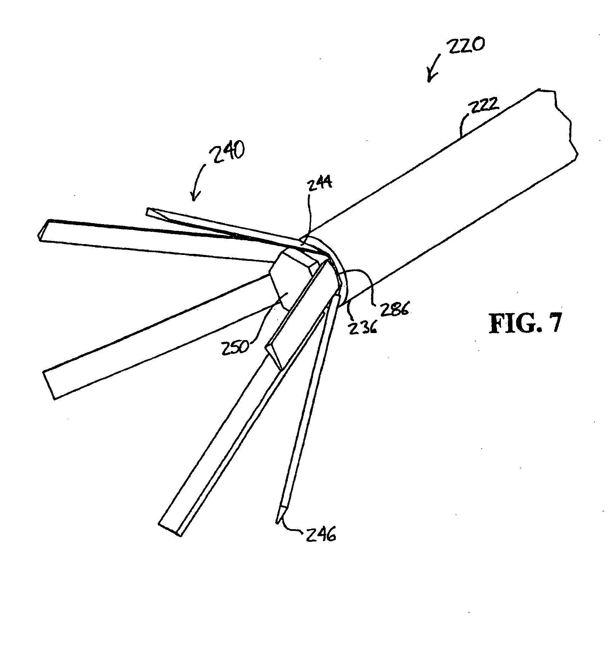

Referring to Fig. 7, third exemplary expandable reamer 220 includes cannulated shaft 222, expandable blades 240, and expansion member 250. In the third exemplary embodiment, blades 240 are coupled at proximate end 244 to distal end 236 of cannulated shaft 222. Deformation points 286 are formed in blades 240 where blades 240 are joined to cannulated shaft 222. In a contracted position, blades 240 are substantially parallel to the longitudinal axis of shaft 222, similar to the arrangement shown in Fig. 1 for first exemplary reamer 20, and expansion member 250 is positioned near distal end 246 of blades 240.

Expansion member 250 has a larger diameter than the interior diameter between circumferentially located blades 240 adjacent proximate end 244; therefore, as expansion member 250 is drawn proximally from distal end 246 to proximal end 244 of blades 240, distal ends 246 extend radially outward about deformation points 286, as shown in Fig. 7. To contract blades 240 to their original position, expansion member 250 may be distally advanced to the original position near distal end 246 of blades 240, thus allowing blades 240 to return to the original positions substantially parallel to shaft 222.

Blades 240 may return to the original position by the force applied by the structure being reamed as reamer 220 is withdrawn from the cavity formed. For example, as reamer 220 is withdrawn from the cavity, blades 240 may contact the structure walls forming the aperature leading into the cavity because blades 240 form a diameter between proximate end 244 and distal end 246 that is greater than the diameter of the aperature. Thus, blades 240 will be deformed to the contracted position as reamer 240 is withdrawn from the cavity and through the aperature. Blades 240 may also be spring loaded or otherwise biased to their original contracted positions. Alternatively, reamer 220 may include engagement devices (not shown) coupled to expansion member 250. The engagement devices draw blades 240 radially inward as member 250 extends distally from proximate end 244 toward distal end 246 of blades 240.

Referring again to Fig. 6, various combinations of the above-disclosed aspects of exemplary reamers 20, 120, and 220 may be utilized, as well as other aspects known in the art, in order to provide an expandable reamer that is well suited for a particular task. For example, various portions of the expandable reamer may be formed from a selected metal, polymer, or other material.

In addition, blades 40, 140, and 240, which are deformable from a contracted position to an expanded position, may be spring loaded or otherwise biased to an expanded position. Return to a contracted position may be controlled by the amount of force applied to the blade surfaces. For example, blades 40 may be normally biased to the expanded position as shown in Fig. 3A. However, application of a force against blades 40 may deform blades 40 to the contracted position, as shown in Fig. 1, if the force is great enough to overcome, for example, spring loading, the material strength of deformation points 86, or another resistance to movement to the contract position. Such an arrangement may be used, for example, as a material-sensitive reamer used to remove softer cancellous bone while leaving generally intact the harder cortical bone. Blades 40, 140, or 240 would not expand or would return to a contracted position upon contacting harder or denser material while remaining in an expanded cutting position while contacting the softer or less dense material.

While this invention has been described as having an exemplary design, the present invention may be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains.

## Claims

1. A reamer comprising:
a shank;
a reamer body having a longitudinal axis; and
a blade formed in said reamer body, said blade deformable between a contracted position and an expanded position.

2. The reamer of claim 1, wherein said shank has a radius measured from said longitudinal axis, said blade in said contracted position extending no further from said longitudinal axis than said radius of said shank.

3. The reamer of claim 1, wherein said shank has a radius measured from said longitudinal axis, said blade in said expanded position extending further from said longitudinal axis than said radius of said shank.

4. The reamer of claim 1, wherein said blade in said contracted position is substantially parallel to said longitudinal axis.

5. The reamer of claim 1, wherein said blade in said expanded position comprises a portion oriented radially outward from said longitudinal axis.

6. The reamer of claim 1, wherein said blade comprises at least one deformation point.

7. The reamer of claim 6, wherein said at least one deformation point comprises an exterior circumferential relief.

8. The reamer of claim 7, wherein said exterior circumferential relief is formed in a proximate end of said blade, said reamer further comprises a ring coupled to a distal end of said blade, and said at least one deformation point further comprises an exterior circumferential relief formed in said distal end of said blade.

9. The reamer of claim 6, wherein said at least one deformation point comprises at least one interior circumferential relief formed in said blade between said proximate end and said distal end.

10. The reamer of claim 6, wherein said at least one deformation point comprises a radially oriented cut in said blade.

11. The reamer of claim 6, wherein said at least one deformation point comprises a thinned region.

12. The reamer of claim 1, wherein said reamer body comprises a shaft having a polygonal cross-section, an edge of said blade being coincident with an apex formed by two adjacent sides of said polygonal reamer body.

13. The reamer of claim 1, further comprising an actuating means for moving said blade between said contracted position and said expanded position.

14. The reamer of claim 13, wherein said shank is cannulated and said actuating means comprises an elongate member connected to said blade, proximate translation of said elongate member moving said blade from said contracted position to said expanded position, and distal translation of said elongate member moving said blade from said expanded position to said contracted position.

15. The reamer of claim 1, further comprising an end cutter secured to a distal end of said reamer body.

16. The reamer of claim 1, wherein said blade is biased to said expanded position and is collapsible to said contracted position upon application of a radially inward force upon said blade.

17. A reamer, comprising:
a shank;
a reamer body having a longitudinal axis;
a blade formed in said reamer body; and
deformation means for deforming said blade between a contracted position and an expanded position.

18. The reamer of claim 17, wherein said shank has a radius measured from said longitudinal axis, said blade in said contracted position extending no further from said longitudinal axis than said radius of said shank.

19. The reamer of claim 17, wherein said shank has a radius measured from said longitudinal axis, said blade in said expanded position extending further from said longitudinal axis than said radius of said shank.

20. The reamer of claim 17, wherein said blade in said contracted position is substantially parallel to said longitudinal axis.

21. The reamer of claim 17, wherein said blade in said expanded position comprises a portion oriented radially outward from said longitudinal axis.

22. The reamer of claim 17, wherein said reamer body comprises a shaft having a polygonal cross-section, an edge of said blade being coincident with an apex formed by two adjacent sides of said polygonal reamer body.

23. The reamer of claim 17, further comprising an actuating means for moving said blade between said contracted position and said expanded position.

24. The reamer of claim 23, wherein said shank is cannulated and said actuating means comprises an elongate member connected to said blade, proximate translation of said elongate member moving said blade from said contracted position to said expanded position, and distal translation of said elongated member moving said blade from said expanded position to said contracted position.

25. The reamer of claim 17, further comprising an end cutter secured to a distal end of said reamer body.

26. The reamer of claim 17, wherein said blade is biased to said expanded position and is collapsible to said contracted position upon application of a radially inward force upon said blade.

27. A reamer, comprising:
a cannulated shaft having a wall, a proximate end and a distal end, said cannulated shaft defining a longitudinal axis, said wall having a plurality of slots therethrough, said plurality of slots extending from said distal end toward said proximate end; and
a plurality of blades, each one of said plurality of blades defined by said wall between adjacent ones of said plurality of slots.

28. The reamer of claim 27, wherein each one of said plurality of blades includes at least one segment, adjacent said segments being arranged lengthwise along each one of said plurality of blades.

29. The reamer of claim 28, further comprising:
a plurality of deformation points coupling adjacent said segments and coupling each one of said plurality of blades to said cannulated shaft;
said plurality of blades deformable between a contracted position and an expanded position;
said plurality of blades being substantially parallel to said longitudinal axis in said contracted position; and
said plurality of blades being deformable at said deformation points to achieve said expanded position.

30. The reamer of claim 29, wherein a portion of said wall of said shaft in which said plurality of blades are formed has a polygonal cross-section.

31. The reamer of claim 27, further comprising an end cutter secured to a distal end of said plurality of blades.
